Europäisches Patentamt

European Patent Office

Office européen des brevets

(11) Publication number: **0 104 619 B1**

# EUROPEAN PATENT SPECIFICATION

(45) Date of publication of patent specification: **04.09.91**  (51) Int. Cl.⁵: **A61B 5/0448**, A61B 5/00

(21) Application number: **83109483.4**

(22) Date of filing: **23.09.83**

(54) **Combination spiral EKG electrode and pH probe.**

(30) Priority: **24.09.82 US 425036**

(43) Date of publication of application:
**04.04.84 Bulletin 84/14**

(45) Publication of the grant of the patent:
**04.09.91 Bulletin 91/36**

(84) Designated Contracting States:
**AT BE CH DE FR GB IT LI LU NL SE**

(56) References cited:
**EP-A- 0 069 113
DE-A- 1 909 882
DE-A- 2 930 663
FR-A- 2 120 787**

**N.T.I.S., no. PB82-970453, 1982, Springfield,
Virginia, US; "Fiber optic pH probe for tissue
measurements"* Whole document * & EP - A
- 73 558**

(73) Proprietor: **ABBOTT LABORATORIES**

**Abbott Park, Illinois 60064(US)**

(72) Inventor: **Hochberg, Howard Martin
14474 156th Ave, NE
Woodinville Washington(US)**
Inventor: **Schmalzbach, Edwin Lewis
74 Pine Drive
Roosevelt New Jersey(US)**
Inventor: **Ziedonis, Janis Gunars
8 Steel Drive
Cranburg New Jersey(US)**

(74) Representative: **Vossius & Partner
Siebertstrasse 4 P.O. Box 86 07 67
W-8000 München 86(DE)**

**Description**

The present invention is an electrode for use in fetal monitoring. In particular, the invention relates to an electrode for use in continuously measuring both EKG signals and hydrogen ion concentration (pH) of a fetus during birth.

Heretofore, a variety of electrode structures and applicators have been described for use in inserting a bipolar electrode structure through the vagina and cervix of a woman in labor and attaching it to the epidermis of a fetus. Such electrode structures are designed to be operatively connected to an amplifier and a cardiotachometer for recording the fetal electrocardiogram and heart rate during labor and delivery.

The pH value of blood is related largely to the carbon dioxide and acid concentration in the blood. For many years it has been recognized that the pH value of the blood (or tissue) of a fetus during delivery may provide an early indication of fetal distress, since increased carbon dioxide, with or without fixed acid concentration (manifested by decreasing pH values), may mean that the fetus is receiving insufficient oxygen. That can be an indication of compression of the umbilical cord, which could result in restricted oxygen supply and permanent brain damage.

Heretofore, various pH electrodes have been developed for the purpose of continuously monitoring the pH values of the fetus. Typically, pH values were measured using two electrochemical half cells with the potentials across the half cells connected in opposition, so as to cancel one another. NTIS, No. PB 82-970453 (1982), Springfield, VA, USA, describes a fiber optic pH probe for tissue measurements incorporated into a small rigid needle.

As noted, the monitoring of the electrocardiograph and the pH of a fetus are both desirable. Accordingly, a number of different types of electrodes have heretofore been developed to monitor either EKG or the pH. Unfortunately, in view of the manner in which such electrodes are applied to the scalp of the fetus, i.e., through the vagina and cervix of a woman in labor, it has not heretofore been possible to attach more than one electrode to the scalp of a fetus at any given time. Therefore, it would be highly desirable to have a combined pH and EKG electrode.

In accordance with the present invention a combined EKG electrode and pH probe comprises a hollow, electrically conductive spiral needle which houses a fiber optic pH probe. The combined unit serves to electrically measure EKG while optically measuring the pH of the fetus.

In the Drawing:

FIG. 1 is a side view of the combined pH and EKG probe of the present invention:

FIG. 2 is a bottom view of the probe of FIG. 1:

FIG. 3 is a cross-sectional side view of the probe of FIG. 1:

FIG. 4 is an exploded view of the preferred embodiment of the end of the needle used in the present invention: and

FIG. 5 is an alternative embodiment for use with the end of the needle.

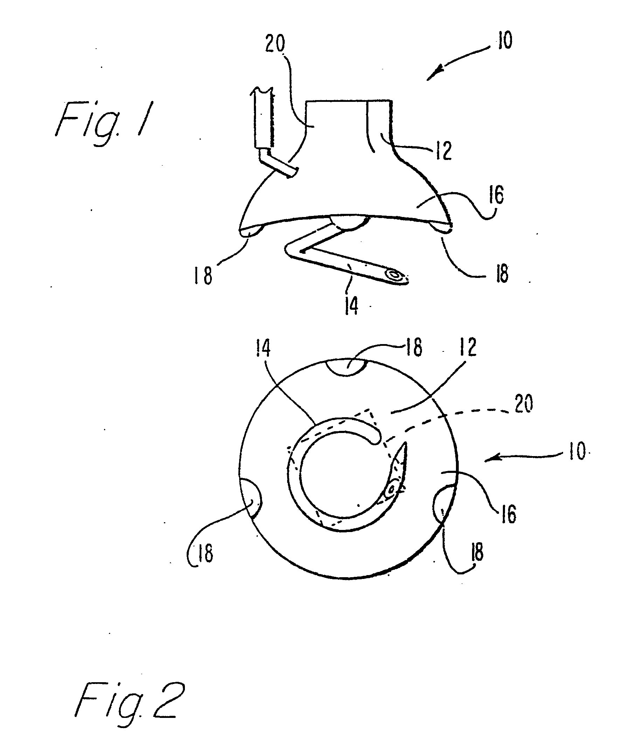

Referring generally to FIGS. 1 - 3, the preferred embodiment of the combined pH and EKG probe 10 of the present invention is shown. The probe 10 is comprised of a base assembly 12 which is made of a soft material such as a silicone plastic. Molded into the plastic base 12 is a hollow, electrically conductive, spiral wound needle 14. The needle 14 is used both as the EKG electrode and also as a housing for the fiber optic probe. The needle 14 extends out of the broad portion 16 of the base assembly 12. The broad portion 16 of the base assembly 12 is somewhat concave and has raised bumps 18 formed thereon. The base assembly 12 tapers from the broad portion 16 to the top 20 which is substantially squared in shape (when viewed from the end) in order that a square tube (not shown) can be used as an applicator for inserting the electrode 10 into scalp of the fetus through the vagina and cervix of a woman in labor. A pair of wires 22, 24 are electrically connected to the hollow, spiral wound needle 14 and to a reference electrode 26 formed at the top 20 of the plastic base assembly 12. The hollow needle 14 extends through the side 28 of the base assembly 12 in order to permit optical fibers 30, 32 to pass down through a tube 34 into the end of the needle 14.

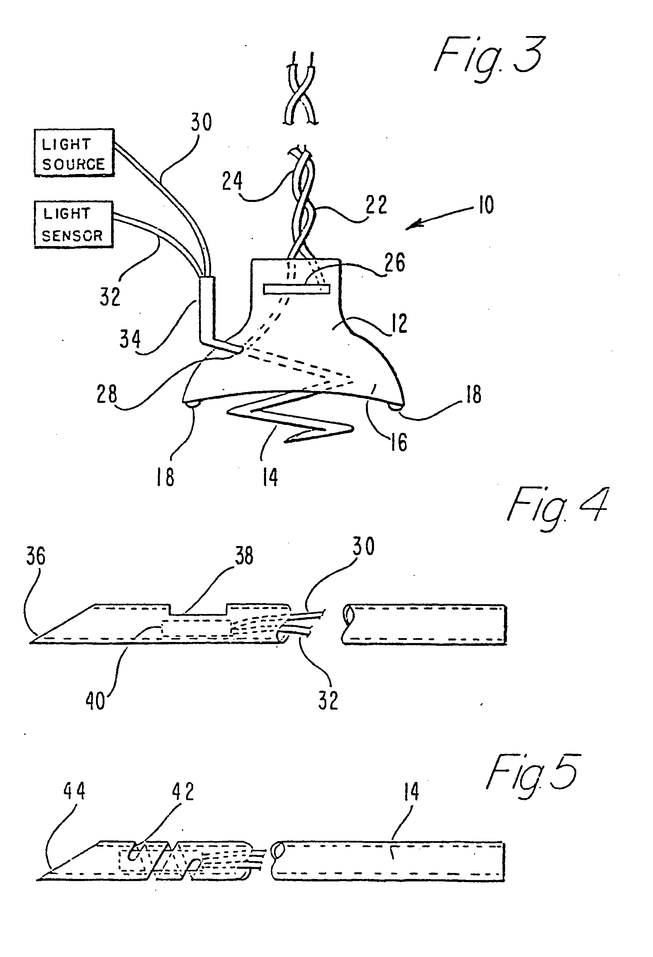

Referring now to FIG. 4, the end of the needle 14 comes to a point 36. Near the point 36 of the needle 14 is a window 38 formed in the needle 14 in order to allow body fluids from the scalp of the fetus to enter into the area containing the fiber optic probe 40 and ion permeable membrane envelope. These items operate in a manner more fully described in U. S. Patent No. 4,200,110 entitled FIBER OPTIC PH PROBE which issued to J.I. Peterson, et al. on April 29, 1980.

Referring to FIG. 5, an alternative embodiment for the end of the needle is shown. In the alternative embodiment there is a spiral opening 42 cut adjacent the end 44 of the needle 14 to permit body fluids from the scalp of the fetus to enter the ion permeable membrane of the fiber optic pH probe.

The purpose of the squared off top end 20 of the plastic assembly 12 is to permit the use of an elongated tube (not shown), which is typically constructed of plastic, which also has a squared off end to be used as an insertion tool for applying the probe 10 through the vagina and cervix of a woman in labor and into the scalp of a fetus.

The present probe 10 provides for simultaneous monitoring of both pH and EKG of a fetus during labor without requiring that more than one probe be inserted into the scalp of the fetus. Thus, the benefits derived from both EKG monitoring and from pH monitoring are simultaneously available.

## Claims

1. A combined EKG electrode and pH probe for simultaneous electrical and chemical monitoring of a fetus, comprising:

   a base assembly (12);
   a single, spiral shaped hollow electrically conductive needle (14)used as an EKG electrode and, which extends from said base assembly (12) for electrical monitoring of a fetus;
   a reference electrode (26) on said base assembly (12); and
   a fiber-optic pH probe (30, 32) which extends through said hollow needle (14).

2. The probe of Claim 1, wherein said base assembly (12) is substantially wider than the diameter of said needle (14).

3. The probe of Claim 1 or 2, wherein the tip of said fiberoptic probe (30, 32) is within the portion of said needle (14) adjacent to the tip (36) thereof.

4. The probe of Claim 3, wherein said needle (14) includes an opening (38) adjacent to the tip (36) thereof which permits fluid from the fetal scalp to contact said fiberoptic probe (30, 32).

5. The probe of Claim 3 or 4, wherein the top of said base assembly (12) has a substantially square shape so as to conveniently receive an insertion tool having the same shape, for ease of insertion of said probe into the scalp of a fetus through the vagina and cervix of a woman in labor.

6. The probe of any of Claims 1 to 5, wherein said base assembly (12) is made of a plastic material.

7. The probe of any of Claims 4 to 6, wherein said opening (38) in said needle (14) is substantially rectangular.

8. The probe of any of Claims 4 to 6, wherein said opening (38) in said needle (14) is a spiral shaped opening.

9. The probe of any of Claims 3 to 8 further comprising means for sending both electrical signals, from said EKG probe, and light signals, from said fiber optic pH probe, to sensing means external to the woman.

## Revendications

1. Electrode ECG combinée avec un capteur de pH destinée au contrôle foetal simultané électrique et chimique sur moniteur, comprenant :
   - un ensemble de base (12) ;
   - une aiguille simple (14) électriquement conductrice, creuse en forme de spirale servant d'électrode ECG et qui s'étend à partir de l'ensemble de base (12) pour le contrôle électrique d'un foetus sur moniteur ;
   - une électrode de référence (26) sur l'ensemble de base (12) ; et
   - un capteur de pH à fibre optique (30, 32) qui s'étend à travers l'aiguille creuse (14).

2. Capteur selon la revendication 1, dans lequel l'ensemble de base (12) est sensiblement plus large que le diamètre de l'aiguille (14).

3. Capteur selon la revendication 1 ou 2, dans lequel la pointe du capteur à fibre optique (30, 32) se situe à l'intérieur de la portion de l'aiguille (14) contiguë à sa pointe (36).

4. Capteur selon la revendication 3, dans lequel l'aiguille (14) comprend une ouverture (38) contiguë à sa pointe (36), qui permet au fluide provenant du crâne foetal de venir en contact avec le capteur à fibre optique (30, 32).

5. Capteur selon la revendication 3 ou 4, dans lequel le sommet de l'ensemble de base (12) présente une forme pratiquement carrée de façon à pouvoir recevoir un instrument d'insertion ayant la même forme pour faciliter l'introduction du capteur sur le crâne d'un foetus par le vagin et le col de l'utérus d'une parturiente.

6. Capteur selon l'une quelconque des revendications 1 à 5, dans lequel l'ensemble de base (12) est réalisé en une matière plastique.

7. Capteur selon l'une quelconque des revendications 4 à 6, dans lequel l'ouverture (38) dans l'aiguille (14) est sensiblement rectangulaire.

8. Capteur selon l'une quelconque des revendications 4 à 6, dans lequel l'ouverture (38) dans l'aiguille (14) est en forme de spirale.

9. Capteur selon l'une quelconque des revendications 3 à 8, comprenant des moyens destinés à émettre à la fois des signaux électriques provenant du capteur ECG et des signaux lumineux en provenance du capteur de pH à fibre optique au dispositif de détection externe à la parturiente.

**Patentansprüche**

1. pH-Sonde in Kombination mit einer EKG-Spiralelektrode für die gleichzeitige elektrische und chemische Überwachung eines Fötus, mit:

   einem Basiskörper (12);

   einer einzelnen, spiralförmigen, elektrisch leitenden Hohlnadel (14), die als eine EKG-Elektrode verwendet wird und sich von dem Basiskörper (12) erstreckt, um einen Fötus elektrisch zu überwachen;

   einer Referenzelektrode (26) auf dem Basiskörper (12); und einer faseroptischen pH-Sonde (30, 32), die sich durch die Hohlnadel (14) erstreckt.

2. Sonde nach Anspruch 1, wobei der Basiskörper (12) im wesentlichen weiter als der Durchmesser der Nadel (14) ist.

3. Sonde nach Anspruch 1 oder 2, wobei die Spitze der faseroptischen Sonde (30, 32) innerhalb des Teils der Nadel (14) angeordnet ist, der an ihre Spitze (36) angrenzt.

4. Sonde nach Anspruch 3, wobei die Nadel (14) eine Öffnung (38) aufweist, die an ihre Spitze (36) angrenzt und einem Fluid aus der Kopfhaut des Fötus gestattet, die faseroptische Sonde (30, 32) zu kontaktieren.

5. Sonde nach Anspruch 3 oder 4, wobei das Oberteil des Basiskörpers (12) eine im wesentlichen quadratische Form aufweist, um so ein Einsetzwerkzeug der gleichen Gestalt passend aufzunehmen und das Einsetzen der Sonde in die Kopfhaut eines Fötus durch die Vagina und den Gebärmutterhals einer Frau in den Wehen zu erleichtern.

6. Sonde nach einem der Ansprüche 1 bis 5, wobei der Basiskörper (12) aus einem Plastikmaterial hergestellt ist.

7. Sonde nach einem der Ansprüche 4 bis 6, wobei die Öffnung (38) in der Nadel (14) im wesentlichen rechteckig ist.

8. Sonde nach einem der Ansprüche 4 bis 6, wobei die Öffnung (38) in der Nadel (14) eine spiralförmige Öffnung ist.

9. Sonde nach einem der Ansprüche 3 bis 8, die ferner Einrichtungen zum Senden sowohl von elektrischen Signalen von der EKG-Sonde als auch von Lichtsignalen von der faseroptischen PH-Sonde zu Sensoren außerhalb der Frau aufweist.

*Fig. 1*

*Fig. 2*

*Fig. 3*

LIGHT SOURCE

LIGHT SENSOR

30

24

22

10

26

32

12

34

28

16

18

18

14

18

*Fig. 4*

36

38

30

32

40

*Fig. 5*

44

42

14